# EUROPEAN PATENT SPECIFICATION

(11) **EP 3 734 603 B1**
(45) Date of publication and mention of the grant of the patent: **17.07.2024**
(21) Application number: 17936153.0
(22) Date of filing: 28.12.2017
(51) Int. Cl.: G16B 15/30, G16C 20/50

(54) **METHOD AND DEVICE FOR COMPUTING STABLE BINDING STRUCTURE AND PROGRAM**
VERFAHREN UND VORRICHTUNG ZUR BERECHNUNG EINER STABILEN BINDUNGSSTRUKTUR UND PROGRAMM
PROCÉDÉ ET DISPOSITIF PERMETTANT DE CALCULER UNE STRUCTURE DE LIAISON STABLE ET PROGRAMME

(43) Date of publication of application: 04.11.2020
(73) Proprietor: FUJITSU LIMITED, Kanagawa 211-8588 (JP)
(72) Inventor: TANIDA, Yoshiaki, Kawasaki-shi, Kanagawa 211-8588 (JP)
(74) Representative: Haseltine Lake Kempner LLP
(86) International application number: PCT/JP2017/047161
(87) International publication number: WO 2019/130529

(56) References cited:
- WO-A1-2016/072027
- ANDREA SPITALERI ET AL: "Use of Metadynamics in the Design of isoDGR-Based [alpha]v&bgr;3 Antagonists To Fine-Tune the Conformational Ensemble", ANGEWANDTE CHEMIE (INTERNATIONAL ED. IN ENGLISH), vol. 123, no. 8, 18 February 2011 (2011-02-18), pages 1872 - 1876, XP055065956, ISSN: 0044-8249, DOI: 10.1002/ange.201007091
- BAI QIFENG ET AL: "Molecular modeling study on the dynamical structural features of human smoothened receptor and binding mechanism of antagonist LY2940680 by metadynamics simulation and free energy calculation", BIOCHIMICA ET BIOPHYSICA ACTA (BBA) - GENERAL SUBJECTS, ELSEVIER, AMSTERDAM, NL, vol. 1840, no. 7, 15 March 2014 (2014-03-15), pages 2128 - 2138, XP028853738, ISSN: 0304-4165, DOI: 10.1016/J.BBAGEN.2014.03.010
- JAMES MCCARTY; MICHELE PARRINELLO: "A variational conformational dynamics approach to the selection of collective variables in metadynamics", ARXIV.ORG, 8 December 2017 (2017-12-08), XP080759655

## Description

### TECHNICAL FIELD

The present invention relates to a method and a device for computing a stable binding structure between a target molecule and a drug candidate molecule, and a program that executes the computing method.

### BACKGROUND ART

In recent years, various simulations have been performed by a calculator in order to reduce enormous cost and labor required for experimentally searching for a drug candidate molecule. The calculation of a drug candidate molecule is to calculate a molecule (ligand) that strongly interacts with a target molecule involved in a target disease (disease as a target) as a drug candidate. Therefore, screening of a compound based on a three-dimensional structure of a target molecule by a calculator is actively performed.

A particularly utilized method is, for example, a structure-based drug design (SBDD) method (for example, see Non-patent Document 1). This method is a molecular design method based on three-dimensional structure information of a target molecule (for example, a protein).

When a stable binding structure (complex structure) between a target molecule and a drug candidate molecule in a solvent is search for using a calculator, it is known that a calculation method using an alchemical thermodynamic cycle is the most accurate. However, in this method, the calculation accuracy depends on the accuracy of the structure. Furthermore, it is necessary to consider structural fluctuations of the target molecule and the drug candidate molecule in the solvent due to heat, and there is no appropriate method. In general, docking simulation is used as a method for predicting a stable binding structure. However, this method ignores structural fluctuations of a target molecule and a drug candidate molecule, and therefore a satisfactory result cannot be obtained. Moreover, in an experiment, X-ray structural analysis is used. However, in this case, it is necessary to crystallize a complex, and information on a case where a plurality of complex structures is established cannot be obtained.

### CITATION LIST

### NON-PATENT DOCUMENT

Non-Patent Document 1: The Process of Structure-Based Drug Design", A.C. Anderson, Chemistry & Biology, 10, 787 (2003).
Non-Patent Document 2: *"*Use of Metadynamics in the Design of isoDGR-Based αvβ3 Antagonists To Fine-Tune the Conformational Ensemble", A. Spitaleri et al., Angewandte Chemie, vol. 123, no. 8, 1872-1876 (2011) discloses a protocol based on the combination of MetaD and docking simulations to analyze the conformations and the αvβ3-binding properties of isoDGR-containing cyclopeptides and to predict the conformational effects of chemical modifications and discriminate binders from nonbinders in silico.
Non-Patent Document 3: *"*Molecular modeling study on the dynamical structural features of human smoothened receptor and binding mechanisms of antagonist LY2940680 by metadynamics simulation and free energy calculation", Q. Bai et al., Biochimica et Biophysica Acta, vol. 1840, no. 7, 2128-2138 (2014) uses molecular dynamics (MD) simulation and dynamical network analysis to study the dynamical structural features of SMO receptor. Metadynamics simulation and free energy calculation are employed to explore the binding mechanism between the antagonist and SMO receptor.

### SUMMARY OF INVENTION

### TECHNICAL PROBLEM

An object of the present invention to solve the above problems in related art and to achieve the following objects. In other words, an object of the present invention is to provide a method and a device for computing a stable binding structure, capable of efficiently computing a plurality of stable binding structures, and a program that executes the computing method.

### SOLUTION TO PROBLEM

Aspects of the invention are set out in the appended claims.

### ADVANTAGEOUS EFFECTS OF INVENTION

According to the disclosed method for computing a stable binding structure, the above problems in related art can be solved, the above object can be achieved, and a plurality of stable binding structures can be efficiently computed.

According to the disclosed program, the above problems in related art can be solved, the above object can be achieved, and a plurality of stable binding structures can be efficiently computed.

According to the disclosed device for computing a stable binding structure, the above problems in related art can be solved, the above object can be achieved, and a plurality of stable binding structures can be efficiently computed.

### BRIEF DESCRIPTION OF DRAWINGS

FIG. 1A is a conceptual diagram of searching a structural space using metadynamics (part 1).
FIG. 1B is a conceptual diagram of searching a structural space using metadynamics (part 2).
FIG. 1C is a conceptual diagram of searching a structural space using metadynamics (part 3).
FIG. 2A is a schematic top view of a binding site of a target molecule.
FIG. 2B is a schematic side view of a binding site of a target molecule.
FIG. 3 is a flowchart of an example of a disclosed method for computing a stable binding structure.
FIG. 4 is a flowchart of another example of the disclosed method for computing a stable binding structure.
FIG. 5 illustrates a configuration example of a disclosed device for computing a stable binding structure.
FIG. 6 illustrates another configuration example of the disclosed device for computing a stable binding structure.
FIG. 7 illustrates another configuration example of the disclosed device for computing a stable binding structure.
FIG. 8 is a schematic diagram of a dihedral angle used in Example.
FIG. 9 is a graph illustrating a free energy surface obtained in Example.

### DESCRIPTION OF EMBODIMENTS

### (Method for computing stable binding structure)

A disclosed method for computing a stable binding structure is a method for computing a stable binding structure between a target molecule and a drug candidate molecule using a computer.

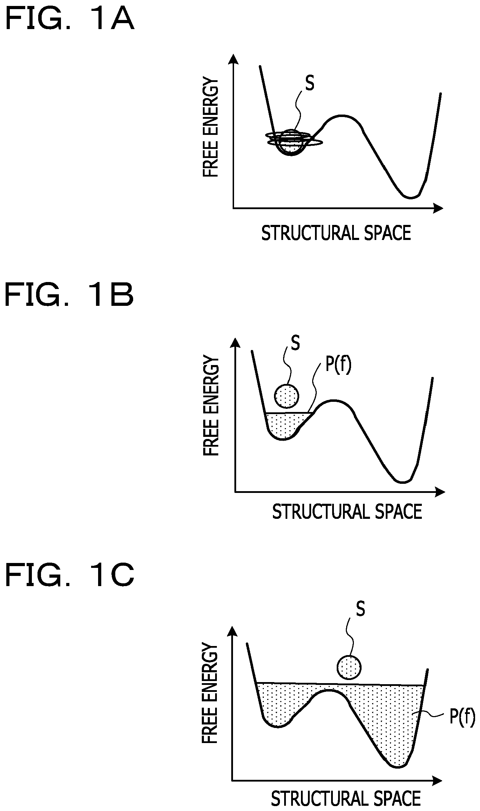

The present inventor studied search for a stable binding structure using metadynamics. As illustrated in FIGS. 1A to 1C, the metadynamics intends to expand an area of a structural space in which binding structure S can be searched for by filling a potential valley with penalty function P (f). Therefore, in the metadynamics, denaturation of a target molecule does not occur unlike simulated annealing in which a simulation temperature is set to be high and a search area is extended to a structural space that cannot be searched by room temperature simulation, and does not break a network of water molecules.

In the metadynamics, a plurality of stable binding structures can be searched for because a structural space to be searched is large.

Here, the metadynamics is one of tabu search methods, and is a method for suppressing an existence probability in an once visited area to achieve a smooth probability distribution on coordinates by placing a potential proportional to the existence probability on coordinate axes (by imparting a penalty function). Note that a Gaussian distribution is often used for the penalty function.

In other words, the metadynamics is a method for smoothing a free energy surface by adding minute potentials one after another to a free energy curved surface (minimum) of a system by a penalty function.

However, when it is intended to use metadynamics in searching for a stable binding structure between a target molecule and a drug candidate molecule, a structural space to be searched is large. Therefore, if usual two collective variables used in searching for the stable binding structure between the target molecule and the drug candidate molecule [distance (for example, a distance between the center of gravity of the target molecule and the center of gravity of the drug candidate molecule), and an angle formed by a line connecting the center of gravity of the target molecule and the center of gravity of the drug candidate molecule and a line connecting the center of gravity of the drug candidate molecule and one of constituent atoms of the drug candidate molecule] are used, calculation time is extremely long disadvantageously.

Here, the collective variable refers to a variable constituting a space to be searched.

In order to solve such a problem, the present inventor uses the following experimental facts and has found the disclosed technology.
(i) Generally, a ligand molecule (drug candidate molecule) has one or more heterocycles.
(ii) In a stable binding structure, the shape of a target molecule in a binding site matches the heterocycle of the ligand molecule.

Then, the present inventor has found that a plurality of stable binding structures can be efficiently searched for by using a specific one collective variable, and has completed the disclosed technology.

The disclosed method for computing a stable binding structure is a method for computing a stable binding structure between a target molecule and a drug candidate molecule having a heterocycle using a computer.

In the method for computing a stable binding structure, the stable binding structure between the target molecule and the drug candidate molecule is searched for using a dihedral angle as a collective variable in a structural space in which the target molecule and the drug candidate molecule at the binding site of the target molecule are disposed when the stable binding structure between the target molecule and the drug candidate molecule is searched for using metadynamics.

The dihedral angle is a dihedral angle formed by plane X formed by the following points A1, A2, and A3 and plane Y formed by the following points A1, A2, and A4.

Point A1 is a point determined by using an area where the heterocycle can exist on a surface of a binding site of the target molecule. When there is a plurality of the areas, the point A1 is a point representing the plurality of areas.

Point A2 is a point representing the heterocycle.

Point A3 is a point representing the binding site.

Point A4 is a point representing the drug candidate molecule.

However, the points A1 and A3 do not overlap each other, the points A2 and A4 do not overlap each other, and vectors A1A2 and A2A3 are linearly independent of vector A2A4.

A method for creating a structural space in which the target molecule and the drug candidate molecule at a binding site of the target molecule are disposed is not particularly limited and can be appropriately selected depending on a purpose. Examples of the method include molecular dynamics simulation of the target molecule and the drug candidate molecule, and the like. The structural space is, for example, a three-dimensional coordinate space. Furthermore, the drug candidate molecule may be manually disposed at a binding site of the target molecule in consideration of hydrogen bond, van der Waals force, and the like. Note that the state in which the target molecule and the drug candidate molecule at a binding site of the target molecule are disposed preferably has a stable binding structure having a minimum value of energy. However, the state does not necessarily need to have a stable binding structure.

The molecular dynamics simulation can be performed using a molecular dynamics calculation program. Examples of the molecular dynamics calculation program include AMBER, CHARMm, GROMACS, GROMOS, NAMD, myPresto, and the like.

The target molecule is not particularly limited and can be appropriately selected depending on a purpose. Examples of the target molecule include a protein, a ribonucleic acid (RNA), a deoxyribonucleic acid (DNA), and the like.

The drug candidate molecule is not particularly limited as long as having a heterocycle, and can be appropriately selected depending on a purpose. The drug candidate molecule may have one heterocycle, or may have two or more heterocycles. Here, when the number of the heterocycles in the drug candidate molecule is counted, a case where two or more rings share two or more atoms and are bound to each other is counted as one. A case where two or more rings are bound to each other via a double bond that is not rotatable about an axis is also counted as one. Meanwhile, for example, a case where two rings are bound to each other via a single bond that is rotatable about an axis is counted as two. For example, the number of the heterocycles in theophylline or purine is one.

Examples of the heterocycle include an aliphatic heterocycle, a 5-membered aromatic heterocycle, a 6-membered aromatic heterocycle, a polycyclic aromatic heterocycle, and the like.

Examples of the aliphatic heterocycle include a piperidine ring, a piperazine ring, a morpholine ring, a quinuclidine ring, a pyrrolidine ring, an azetidine ring, an oxetane ring, an azetidin-2-one ring, an aziridine ring, a tropane ring, and the like.

Examples of the 5-membered aromatic heterocycle include a pyrrole ring, a furan ring, a thiophene ring, an imidazole ring, a pyrazole ring, an oxazole ring, an isoxazole ring, a thiazole ring, an isothiazole ring, and the like.

Examples of the 6-membered aromatic heterocycle include a pyridine ring, a pyrimidine ring, a pyridazine ring, a pyrazine ring, a 1,2,3-triazine ring, and the like.

Examples of the polycyclic aromatic heterocycle include a quinoline ring, an isoquinoline ring, a quinazoline ring, a phthalazine ring, a pteridine ring, a coumarin ring, a chromone ring, a 1,4-benzodiazepine ring, an indole ring, a benzimidazole ring, a benzofuran ring, a purine ring, an acridine ring, a phenoxazine ring, a phenothiazine ring, and the like.

Search for the stable binding structure is preferably performed in a solvent, more preferably in water. This makes it possible to obtain a stable binding structure closer to a binding structure in a living body.

When the metadynamics is used for computing a stable binding structure between a target molecule and a drug candidate molecule using a computer, a specific method of the metadynamics is not particularly limited and can be appropriately selected depending on a purpose. For example, the metadynamics can be performed with reference to document (Francesco Luigi Gervasio, Alessandro Laio, and Michele Parrinello, J. AM. CHEM. SOC. 2005, 127, 2600-2607).

Search for the stable binding structure using the metadynamics can be performed, for example, by imparting a penalty function (penalty potential) to potential energy of a binding structure in a snapshot in molecular dynamics simulation of the target molecule and the drug candidate molecule. At this time, the dihedral angle is used as a collective variable.

The penalty function is not particularly limited and can be appropriately selected depending on a purpose, but is usually a Gaussian distribution type function.

The width and height in parameters of the penalty function are not particularly limited and can be appropriately selected depending on a purpose.

The penalty function is usually imparted a plurality of times. A frequency (time interval) for imparting the penalty function is not particularly limited and can be appropriately selected depending on a purpose. In other words, the penalty function may be imparted for each time step in the molecular dynamics simulation, or the penalty function may be imparted for each unit including several time steps. At this time, parameters of the penalty function used are preferably fixed.

A method for determining a binding site of the target molecule is not particularly limited and can be appropriately selected depending on a purpose. For example, when the binding site of the target molecule is known, the binding site may be used. When a target molecule whose binding site is unknown is used, the binding site may be appropriately determined by observing a surface of the target molecule from the three-dimensional structure of the target molecule. For example, a site having many depressions on a surface of the three-dimensional structure of the target molecule may be used as a binding site of the target molecule.

An area where the heterocycle can exist on the surface of the binding site of the target molecule is, for example, a depression in the binding site. Here, the depression generally means a shape drawn by a surface when each atom is described by a van der Waals radius. The heterocycle easily interacts with the depression, and the heterocycle easily exist in the depression.

The number of the areas may be one or two or more in the binding site.

The point A1 is a point determined using the area. The point A1 is, for example, the center of gravity of the area. When there is a plurality of the areas in the binding site, the point A1 is determined, for example, as the center of gravity of all heavy atoms in the plurality of areas.

The center of gravity of the area is determined using, for example, heavy atoms existing on a surface of the area.

In the disclosed technology, the center of gravity is determined from the mass of a heavy atom which is an atom other than a hydrogen atom and a position vector.

The point A2 is a point representing the heterocycle. The point A2 is, for example, the center of gravity of the heterocycle.

The point A3 is a point representing the binding site. The point A3 is, for example, the center of gravity of the binding site. Here, the binding site can be generally searched for by describing each atom with a van der Waals radius. Then, the center of gravity of the binding site is determined, for example, using a heavy atom existing on a surface of the binder.

The point A4 is a point representing the drug candidate molecule. The point A4 has, for example, coordinates of an arbitrary heavy atom of the drug candidate molecule. Furthermore, for example, the point A4 may be the center of gravity of the drug candidate molecule.

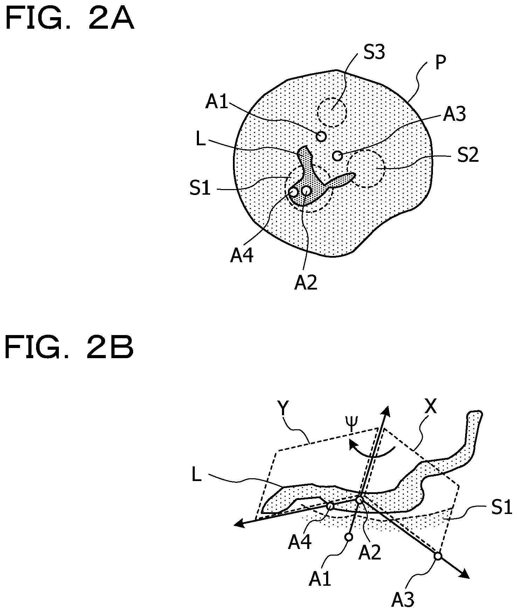

An example of a method for determining the dihedral angle which is the collective variable will be described with reference to FIGS. 2A and 2B.

FIG. 2A is a schematic top view of a binding site of a target molecule.

FIG. 2B is a schematic side view of a binding site of a target molecule.

In this example, as illustrated in FIG. 2A, there are three areas of S1, S2, and S3 in which a heterocycle can exist in a binding site P of a target molecule. The areas S1, S2, and S3 are, for example, depressions in the binding site P. A drug candidate molecule L is bound to a target molecule using these three depressions (areas S1, S2, and S3). The center of gravity of heavy atoms in these three depressions (areas S1, S2, and S3) is referred as point A1.

Next, a case where a heterocycle of the drug candidate molecule L is in the area S1 will be considered. Then, the center of gravity of the heterocycle of the drug candidate molecule L is referred as point A2, and the center of gravity of the binding site P is referred as point A3. Moreover, a constituent atom of the drug candidate molecule L in a direction of a vector that is linearly independent of vectors A1A2 and A2A3 is referred as A4. Then, a dihedral angle Ψ formed by A1-A2-A3 plane X and A1-A2-A4 plane Y is used as a collective variable when a stable binding structure is searched for. By determining the collective variable in this manner, the areas S1, S2, and S3 can be searched for within a search range of a structural space defined by the collective variable. Furthermore, regardless of the stable binding structure, by determining the representative points A1, A2, A3, and A4 by the method described in detail here, search using only one collective variable can be performed.

Note that even if only the dihedral angle Ψ is used as a collective variable as described above, the drug candidate molecule L itself can move in the structural space. Therefore, the drug candidate molecule L can move from the area S1 to the area S2, or can move from the area S2 to the area S3. Furthermore, since the drug candidate molecule is rotatable about an A1A2 line as a rotation axis (in other words, rotatable approximately about a heterocycle involved in determination of A2), a fitting state in each area can be searched for also by considering rotation.

The method for computing a stable binding structure can be implemented by using a general computer system including, for example, a central processing unit (CPU), a random access memory (RAM), a hard disk, various peripheral devices, and the like (for example, various network servers, workstations, personal computers, and the like).

Here, an example of the disclosed method for computing a stable binding structure will be described using a flowchart.

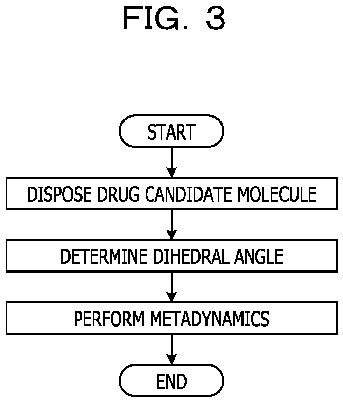

FIG. 3 is a flowchart of an example of the disclosed method for computing a stable binding structure.

First, a drug candidate molecule is disposed at a binding site of a target molecule.

The disposition can be performed, for example, by disposing a drug candidate molecule at a binding site of a target molecule in a coordinate space. As a disposition method, for example, using three-dimensional structure data of a target molecule and three-dimensional structure data of a drug candidate molecule, a three-dimensional structure of the target molecule and a three-dimensional structure of the drug candidate molecule are constructed in a three-dimensional coordinate space. Moreover, when metadynamics is performed in a solvent molecule, a three-dimensional structure of the solvent molecule is constructed in the three-dimensional coordinate space using three-dimensional structure data of the solvent molecule.

The three-dimensional structure data includes, for example, atomic information data, coordinate information data, and binding information data.

A format of the data is not particularly limited, can be appropriately selected depending on a purpose, and for example, may be text data, a structure data file (SDF) format, or a MOL file format.

Subsequently, a dihedral angle is determined. The dihedral angle is determined as a dihedral angle formed by plane X formed by points A1, A2, and A3 and plane Y formed by points A1, A2, and A4.

Here, points A1, A2, A3, and A4 are as follows.

Point A1 is a point determined using an area where a heterocycle can exist on a surface of a binding site of a target molecule.

Point A2 is a point representing the heterocycle.

Point A3 is a point representing the binding site.

Point A4 is a point representing the drug candidate molecule.

However, points A1 and A3 do not overlap each other, points A2 and A4 do not overlap each other, and vectors A1A2 and A2A3 are linearly independent of vector A2A4.

Subsequently, search for a stable binding structure by a metadynamics method is performed using only the determined dihedral angle as a collective variable.

As described above, a stable binding structure is determined.

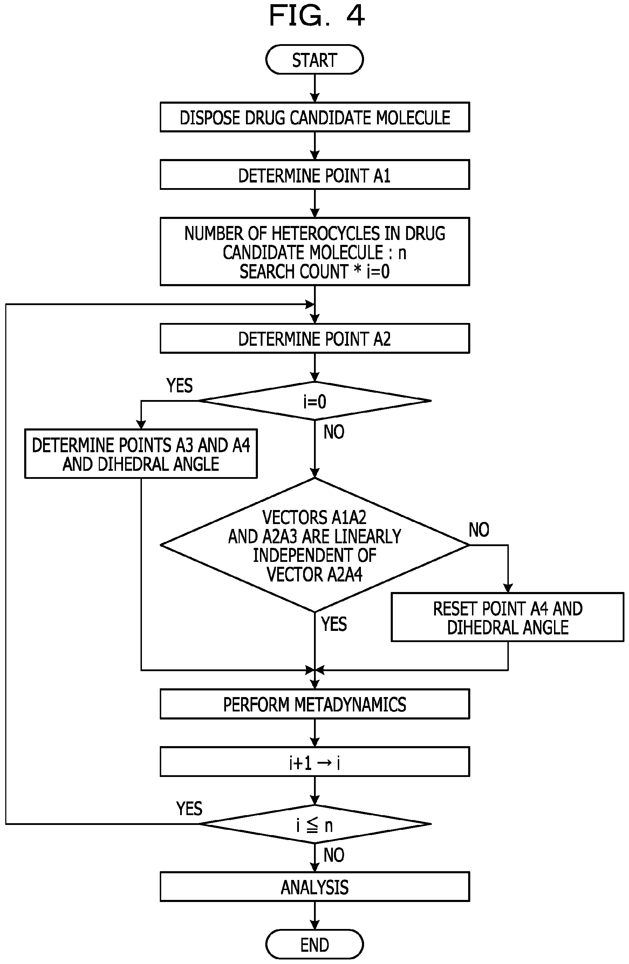

FIG. 4 is a flowchart of another example of the disclosed method for computing a stable binding structure.

The method according to the flowchart in FIG. 4 is an effective method when the drug candidate molecule has two or more heterocycles.

First, a drug candidate molecule is disposed at a binding site of a target molecule.

This can be performed, for example, by disposing the drug candidate molecule at the binding site of the target molecule in a coordinate space. As a disposition method, for example, using three-dimensional structure data of a target molecule and three-dimensional structure data of a drug candidate molecule, a three-dimensional structure of the target molecule and a three-dimensional structure of the drug candidate molecule are constructed in a three-dimensional coordinate space. Moreover, when metadynamics is performed in a solvent molecule, a three-dimensional structure of the solvent molecule is constructed in the three-dimensional coordinate space using three-dimensional structure data of the solvent molecule.

The three-dimensional structure data includes, for example, atomic information data, coordinate information data, and binding information data.

A format of the data is not particularly limited, can be appropriately selected depending on a purpose, and for example, may be text data, a structure data file (SDF) format, or a MOL file format.

Subsequently, point A1 is determined. Point A1 is determined using an area where a heterocycle can exist on a surface of a binding site of the target molecule.

Subsequently, the number of heterocycles in the drug candidate molecule is set as n. An initial value of a search count value i is 0.

Subsequently, point A2 is determined. Point A2 is a point representing the heterocycle.

Subsequently, since the search count value is 0, points 3A and A4 are determined. At this time, a dihedral angle formed by plane X formed by points A1, A2, and A3 and plane Y formed by points A1, A2, and A4 is also determined.

However, the points are determined such that points A1 and A3 do not overlap each other, points A2 and A4 do not overlap each other, and vectors A1A2 and A2A3 are linearly independent of vector A2A4.

Subsequently, search for a stable binding structure by a metadynamics method is performed using only the determined dihedral angle as a collective variable.

Subsequently, 1 is added to the search count value i.

Then, when i ≤ n is satisfied, point A2 is determined for a different heterocycle of the drug candidate molecule.

Subsequently, when the search count value i is 1 or more, it is determined whether vectors A1A2 and A2A3 are linearly independent of vector A2A4.

When vectors A1A2 and A2A3 are linearly independent of vector A2A4, point A4 is not changed, and a stable binding structure is searched for by a metadynamics method using only the determined dihedral angle as a collective variable.

Meanwhile, when vectors A1A2 and A2A3 are not linearly independent of vector A2A4, point A4 is reset. By this reset, the dihedral angle is also reset. Then, a stable binding structure is searched for by a metadynamics method using only the reset dihedral angle as a collective variable.

Subsequently, 1 is added to the search count value i.

Then, when i ≤ n is satisfied, point A2 is determined for a different heterocycle of the drug candidate molecule.

Meanwhile, when i > n is satisfied, the obtained data is analyzed to determine a stable binding structure.

### (Program)

The disclosed program is a program that causes a computer to execute the disclosed method for computing a stable binding structure.

A preferred aspect in the execution of the method for computing a stable binding structure is the same as a preferred aspect in the method for computing a stable binding structure.

The program can be created using various known programming languages according to the configuration of a computer system used, the type and version of an operating system, and the like.

The program may be recorded on a recording medium such as an internal hard disk or an external hard disk, or a recording medium such as a compact disc read only memory (CD-ROM), a digital versatile disk read only memory (DVD-ROM), a magneto-optical disk (MO disk), or a universal serial bus (USB) memory (USB flash drive). When the program is recorded on a recording medium such as a CD-ROM, a DVD-ROM, an MO disk, or a USB memory, through a recording medium reading device of a computer system, the program can be used directly, or can be installed on a hard disk and used at any time as necessary. Furthermore, the program can be recorded in an external storage area accessible from a computer system through an information communication network (another computer and the like), and through the information communication network from the external storage area, the program can be used directly, or can be installed on a hard disk and used at any time as necessary.

The program may be divided and recorded on a plurality of recording media for each arbitrary process.

### (Computer-readable recording medium)

A disclosed computer-readable recording medium records the disclosed program.

The computer-readable recording medium is not particularly limited and can be appropriately selected depending on a purpose. Examples of the computer-readable recording medium include an internal hard disk, an external hard disk, a CD-ROM, a DVD-ROM, an MO disk, a USB memory, and the like.

As the recording medium, a plurality of recording media on which the program is divided and recorded for each arbitrary process may be used. The recording medium may be reversible or irreversible.

### (Device for computing stable binding structure)

The disclosed device for computing a stable binding structure includes at least a search unit, and further includes other units as necessary.

In the search unit, the stable binding structure between the target molecule and the drug candidate molecule is searched for using a dihedral angle as a collective variable in a structural space in which the target molecule and the drug candidate molecule at the binding site of the target molecule are disposed when the stable binding structure between the target molecule and the drug candidate molecule is searched for using metadynamics.

The dihedral angle is a dihedral angle formed by plane X formed by the points A1, A2, and A3 and plane Y formed by the points A1, A2, and A4.

The point A1 is a point determined using an area where the heterocycle can exist on a surface of a binding site of the target molecule. When there is a plurality of the areas, the point A1 is a point representing the plurality of areas.

The point A2 is a point representing the heterocycle.

The point A3 is a point representing the binding site.

The point A4 is a point representing the drug candidate molecule.

However, the points A1 and A3 do not overlap each other, the points A2 and A4 do not overlap each other, and vectors A1A2 and A2A3 are linearly independent of vector A2A4.

As the computing device, a plurality of computing devices each including a plurality of recording media on which the program is divided and recorded for each arbitrary process may be used.

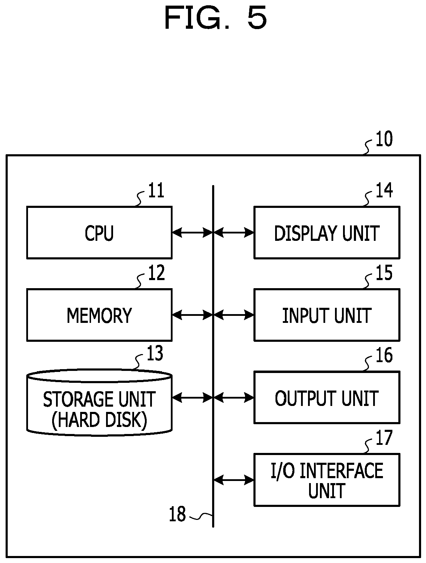

FIG. 5 illustrates a configuration example of a disclosed device for searching for a stable binding structure.

A device 10 for searching for a stable binding structure includes, for example, a CPU 11, a memory 12, a storage unit 13, a display unit 14, an input unit 15, an output unit 16, an I/O interface unit 17, and the like connected via a system bus 18.

The central processing unit (CPU) 11 performs operations (four arithmetic operations, comparison operations, and the like), operation control of hardware and software, and the like.

The memory 12 is a memory such as a random access memory (RAM) or a read only memory (ROM). The RAM stores an operating system (OS), an application program, and the like read from the ROM and the storage unit 13 and functions as a main memory and a work area of the CPU 11.

The storage unit 13 is a device that stores various programs and data, and is for example, a hard disk. The storage unit 13 stores a program executed by the CPU 11, data necessary for executing the programs, an OS, and the like.

The program is stored in the storage unit 13, loaded into a RAM (main memory) of the memory 12, and executed by the CPU 11.

The display unit 14 is a display device, and is for example, a display device such as a CRT monitor or a liquid crystal panel.

The input unit 15 is an input device for various types of data, and is for example, a keyboard, a pointing device (for example, a mouse and the like), or the like.

The output unit 16 is an output device for various types of data, and is for example, a printer.

The I/O interface unit 17 is an interface for connecting various external devices. For example, the I/O interface unit 17 makes input/output of data of a CD-ROM, a DVD-ROM, an MO disk, a USB memory, and the like possible.

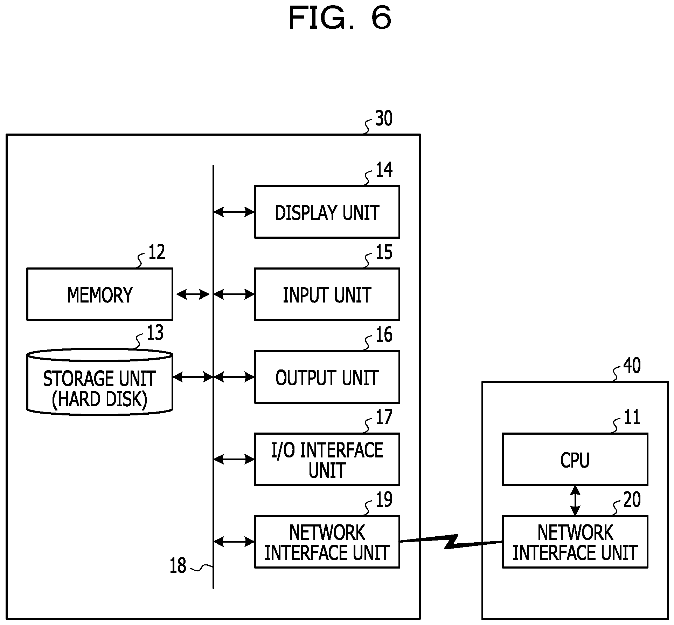

FIG. 6 illustrates another configuration example of the disclosed device for searching for a stable binding structure.

The configuration example in FIG. 6 is a cloud type configuration example, in which the CPU 11 is independent of the storage unit 13 and the like. In this configuration example, a computer 30 that stores the storage unit 13 and the like and a computer 40 that stores the CPU 11 are connected to each other via network interface units 19 and 20.

The network interface units 19 and 20 are hardware that performs communication using the Internet.

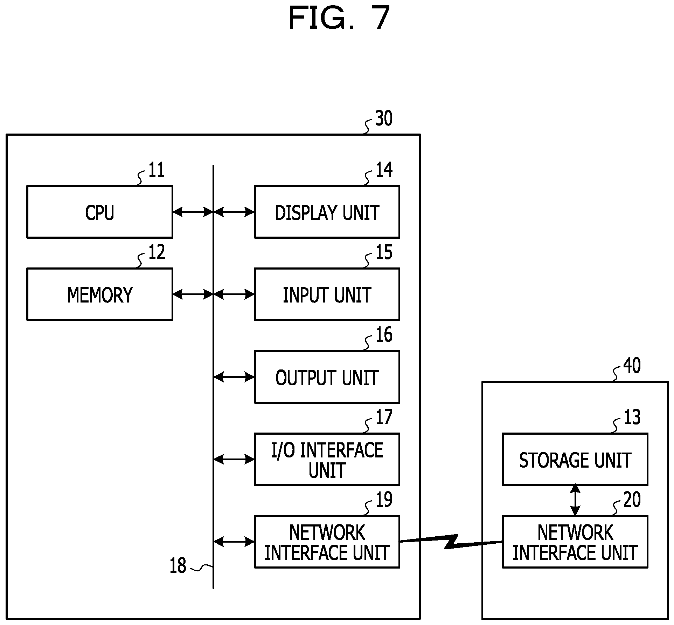

FIG. 7 illustrates another configuration example of the disclosed device for searching for a stable binding structure.

The configuration example in FIG. 7 is a cloud type configuration example, in which the storage unit 13 is independent of the CPU 11 and the like. In this configuration example, the computer 30 that stores the CPU 11 and the like and the computer 40 that stores the storage unit 13 are connected to each other via the network interface units 19 and 20.

The display unit 14 is a display device, and is for example, a display device such as a CRT monitor or a liquid crystal panel.

The input unit 15 is an input device for various types of data, and is for example, a keyboard, a pointing device (for example, a mouse and the like), or the like.

The output unit 16 is an output device for various types of data, and is for example, a printer.

The I/O interface unit 17 is an interface for connecting various external devices. For example, the I/O interface unit 17 makes input/output of data of a CD-ROM, a DVD-ROM, an MO disk, a USB memory, and the like possible.

### EXAMPLE

Hereinafter, the disclosed technology will be described, but the disclosed technology is not limited to the following Examples.

### (Example 1)

An RNA was used as a target molecule, and theophylline was used as a drug candidate molecule. An experimental value of binding free energy of a binding structure (complex) between the RNA and theophylline is -8.9 kcal/mol [see PDB ID: 1015 in Protein Data Bank (PDB)]. Theophylline has one hetero-fused ring as a heterocycle.

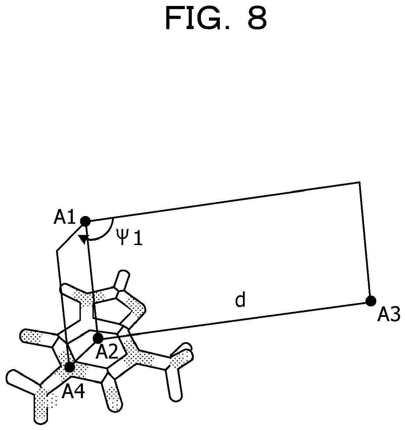

According to the flow illustrated in FIG. 3, the disclosed method for searching for a stable binding structure was performed. Theophylline was disposed on a surface of a binding site of the RNA, and then a dihedral angle W1 was set as illustrated in FIG. 8. Here, points A1 to A4 illustrated in FIG. 8 are as follows. d represents a distance between points A2 and A3.

Point A1 is the center of gravity of heavy atoms of amino acid residues A7, C8, and G26 of the RNA.

Point A2 is the center of gravity of heavy atoms of a heterocycle of theophylline.

Point A3 is the center of gravity of heavy atoms of amino acid residues C22 and U24 of the RNA.

Point A4 has a coordinate at which one of atoms constituting the theophylline ring is located.

Using the above-set dihedral angle W1 as a collective variable, a stable binding structure between the RNA as a target molecule and theophylline as a drug candidate molecule was determined by molecular dynamics simulation using a metadynamics method.

For calculation, a computer equipped with an 80-core CPU [E5-2697A V4 (2.6 GHz)] was used. As a result, the calculation was completed in five days. Here, it was determined that a time when the drug candidate molecule became movable freely in a search space was the completion time.

Note that the calculation takes about three months in related art in which the same computer is used and two collective variables of a distance and an angle are used.

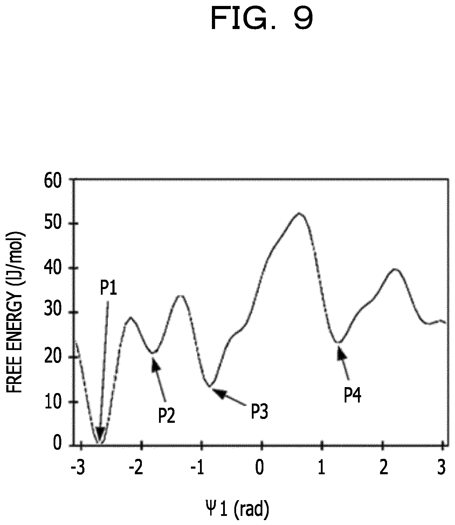

FIG. 9 illustrates a free energy surface obtained by the calculation. In FIG. 9, minimum values were observed at four locations (P1, P2, P3, and P4) relating to a stable binding structure. Binding free energy was determined for these locations. Results are illustrated in Table 1. A calculation result of the binding free energy determined from the results in Table 1 was -9.1 kcal/mol (0.41: statistical error), which was in good agreement with an experimental value of -8.9 kcal/mol. In other words, an accurate calculation result was obtained in a short time.

**[Table 1]**

| Pose | ΔG° _{bind} |
|---|---|
| P1 | -8.92 (0.52) |
| P2 | -7.69 (0.59) |
| P3 | -7.78 (0.47) |
| P4 | -6.34 (0.74) |

The unit of the numerical values in Table 1 is kcal/mol. Values in parentheses are statistical errors due to a bootstrap method.

### REFERENCE SIGNS LIST

- 10: Device for searching for stable binding structure
- 11: CPU
- 12: Memory
- 13: Storage unit
- 14: Display unit
- 15: Input unit
- 16: Output unit
- 17: I/O interface unit
- 18: System bus

## Claims

1. A method for computing a stable binding structure between a target molecule and a drug candidate molecule having a heterocycle using a computer, the method comprising:
arranging, when constructing a three-dimensional structure of the target molecule and a three-dimensional structure of the drug candidate molecule in a three-dimensional coordinate space by using three-dimensional structure data of the target molecule and three-dimensional structure data of the drug candidate molecule, the target molecule and the drug candidate molecule at a binding site of the target molecule;
determining, as a point A1, a point using an area which is a depression in the binding site of the target molecule and where the heterocycle of the drug candidate molecule exists on a surface of the binding site of the target molecule, wherein the point A1 is a center of gravity of the area;
determining, as a point A2, a center of gravity of the heterocycle;
determining, as a point A3, a point which is a center of gravity of the binding site of the target molecule and is different from A1;
determining, as a point A4, a point which is a coordinate of an arbitrary heavy atom of the drug candidate molecule and is different from the A2;
determining, as a collective variable, a dihedral angle formed by a plane X formed by the points A1, A2, and A3 and a plane Y formed by the points A1, A2, and A4; and
searching for the stable binding structure between the target molecule and the drug candidate molecule by using the collective variable using metadynamics,
wherein vectors A1A2 and A2A3 are linearly independent of vector A2A4.

2. A program that, when executed by a computer, causes the computer to carry out the method of claim 1.

3. A device for computing a stable binding structure, the device comprising:
a search unit configured to:
arrange, when constructing a three-dimensional structure of a target molecule and a three-dimensional structure of a drug candidate molecule having a heterocycle in a three-dimensional coordinate space by using three-dimensional structure data of the target molecule and three-dimensional structure data of the drug candidate molecule, the target molecule and the drug candidate molecule at a binding site of the target molecule;
determine, as a point A1, a point using an area which is a depression in the binding site of the target molecule and where the heterocycle of the drug candidate molecule exists on a surface of the binding site of the target molecule, wherein the point A1 is a center of gravity of the area;
determine, as a point A2, a center of gravity of the heterocycle;
determine, as a point A3, a point which is a center of gravity of the binding site of the target molecule and is different from A1;
determine, as a point A4, a point which is a coordinate of an arbitrary heavy atom of the drug candidate molecule and is different from the A2;
determine, as a collective variable, a dihedral angle formed by a plane X formed by the points A1, A2, and A3 and a plane Y formed by the points A1, A2, and A4; and
search for the stable binding structure between the target molecule and the drug candidate molecule by using the collective variable using metadynamics,
wherein vectors A1A2 and A2A3 are linearly independent of vector A2A4.

## Patentansprüche

1. Verfahren zum Berechnen einer stabilen Bindungsstruktur zwischen einem Zielmolekül und einem Arzneimittelkandidatenmolekül mit einem Heterozyklus unter Verwendung eines Computers, wobei das Verfahren umfasst:
Anordnen, wenn eine dreidimensionale Struktur des Zielmoleküls und eine dreidimensionale Struktur des Arzneimittelkandidatenmoleküls in einem dreidimensionalen Koordinatenraum unter Verwendung von dreidimensionalen Strukturdaten des Zielmoleküls und dreidimensionalen Strukturdaten des Arzneimittelkandidatenmoleküls konstruiert werden, des Zielmoleküls und des Arzneimittelkandidatenmoleküls an einer Bindungsstelle des Zielmoleküls;
Bestimmen eines Punkts als Punkt A1 unter Verwendung eines Bereichs, der eine Vertiefung in der Bindungsstelle des Zielmoleküls darstellt und wo der Heterozyklus des Arzneimittelkandidatenmoleküls auf einer Fläche der Bindungsstelle des Zielmoleküls vorhanden ist, wobei der Punkt A1 ein Schwerpunkt des Bereichs ist;
Bestimmen eines Schwerpunkts des Heterozyklus als Punkt A2;
Bestimmen eines Punkts als Punkt A3, wobei der Punkt einen Schwerpunkt der Bindungsstelle des Zielmoleküls darstellt und sich von A1 unterscheidet;
Bestimmen eines Punkts als Punkt A4, wobei der die Koordinate eines schweren Atoms des Arzneimittelkandidatenmoleküls ist und sich von A2 unterscheidet;
Bestimmen eines Flächenwinkels, der von einer durch die Punkte A1, A2 und A3 gebildeten Ebene X und einer durch die Punkte A1, A2 und A4 gebildeten Ebene Y gebildet wird, als kollektive Variable; und
Suchen nach der stabilen Bindungsstruktur zwischen dem Zielmolekül und dem Arzneimittelkandidatenmolekül unter Verwendung der kollektiven Variablen unter Verwendung von Metadynamik,
wobei die Vektoren A1A2 und A2A3 linear unabhängig vom Vektor A2A4 sind.

2. Programm, das bei Ausführung durch einen Computer den Computer dazu veranlasst, das Verfahren nach Anspruch 1 durchzuführen.

3. Vorrichtung zum Berechnen einer stabilen Bindungsstruktur, wobei die Vorrichtung umfasst:
eine Sucheinheit, die zu Folgendem konfiguriert ist:
Anordnen, wenn eine dreidimensionale Struktur eines Zielmoleküls und eine dreidimensionale Struktur eines Arzneimittelkandidatenmoleküls mit einem Heterozyklus in einem dreidimensionalen Koordinatenraum unter Verwendung von dreidimensionalen Strukturdaten des Zielmoleküls und dreidimensionalen Strukturdaten des Arzneimittelkandidatenmoleküls konstruiert werden, des Zielmoleküls und des Arzneimittelkandidatenmoleküls an einer Bindungsstelle eines Zielmoleküls;
Bestimmen eines Punkts als Punkt A1 unter Verwendung eines Bereichs, der eine Vertiefung in der Bindungsstelle des Zielmoleküls darstellt und wo der Heterozyklus des Arzneimittelkandidatenmoleküls auf einer Fläche der Bindungsstelle des Zielmoleküls vorhanden ist, wobei der Punkt A1 ein Schwerpunkt des Bereichs ist;
Bestimmen eines Schwerpunkts des Heterozyklus als Punkt A2;
Bestimmen eines Punkts als Punkt A3, wobei der Punkt einen Schwerpunkt der Bindungsstelle des Zielmoleküls darstellt und sich von A1 unterscheidet;
Bestimmen eines Punkts als Punkt A4, wobei der Punkt die Koordinate eines schweren Atoms des Arzneimittelkandidatenmoleküls ist und sich von A2 unterscheidet;
Bestimmen eines Flächenwinkels, der von einer durch die Punkte A1, A2 und A3 gebildeten Ebene X und einer durch die Punkte A1, A2 und A4 gebildeten Ebene Y gebildet wird, als kollektive Variable; und
Suchen nach der stabilen Bindungsstruktur zwischen dem Zielmolekül und dem Arzneimittelkandidatenmolekül unter Verwendung der kollektiven Variablen unter Verwendung von Metadynamik,
wobei die Vektoren A1A2 und A2A3 linear unabhängig vom Vektor A2A4 sind.

## Revendications

1. Procédé permettant de calculer une structure de liaison stable entre une molécule cible et une molécule candidate médicamenteuse comprenant un hétérocycle à l'aide d'un ordinateur, le procédé comprenant :
l'organisation, lors de la construction d'une structure tridimensionnelle de la molécule cible et d'une structure tridimensionnelle de la molécule candidate médicamenteuse, dans un espace de coordonnées tridimensionnelles à l'aide de données de structure tridimensionnelles de la molécule cible et de données de structure tridimensionnelles de la molécule candidate médicamenteuse, de la molécule cible et de la molécule candidate médicamenteuse au niveau d'un site de liaison de la molécule cible ;
la détermination, en tant que point A1, d'un point en utilisant une zone qui est une dépression dans le site de liaison de la molécule cible et où l'hétérocycle de la molécule candidate médicamenteuse existe sur une surface du site de liaison de la molécule cible, dans lequel le point A1 est un centre de gravité de la zone ;
la détermination, en tant que point A2, d'un centre de gravité de l'hétérocycle ;
la détermination, en tant que point A3, d'un point qui est un centre de gravité du site de liaison de la molécule cible et qui est différent de A1 ;
la détermination, en tant que point A4, d'un point qui est une coordonnée d'un atome lourd arbitraire de la molécule candidate médicamenteuse et qui est différent de A2 ;
la détermination, en tant que variable collective, d'un angle dièdre formé par un plan X formé par les points A1, A2 et A3 et un plan Y formé par les points A1, A2 et A4 ; et
la recherche de la structure de liaison stable entre la molécule cible et la molécule candidate médicamenteuse en utilisant la variable collective utilisant la métadynamique,
dans lequel les vecteurs A1A2 et A2A3 sont linéairement indépendants du vecteur A2A4.

2. Programme qui, lorsqu'il est exécuté par un ordinateur, amène l'ordinateur à exécuter le procédé selon la revendication 1.

3. Dispositif permettant de calculer une structure de liaison stable, le dispositif comprenant :
une unité de recherche configurée pour :
organiser, lors de la construction d'une structure tridimensionnelle d'une molécule cible et d'une structure tridimensionnelle d'une molécule candidate médicamenteuse comprenant un hétérocycle, dans un espace de coordonnées tridimensionnelles à l'aide de données de structure tridimensionnelles de la molécule cible et de données de structure tridimensionnelles de la molécule candidate médicamenteuse, de la molécule cible et de la molécule candidate médicamenteuse au niveau d'un site de liaison de la molécule cible ;
déterminer, en tant que point A1, un point en utilisant une zone qui est une dépression dans le site de liaison de la molécule cible et où l'hétérocycle de la molécule candidate médicamenteuse existe sur une surface du site de liaison de la molécule cible, dans lequel le point A1 est un centre de gravité de la zone ;
déterminer, en tant que point A2, un centre de gravité de l'hétérocycle ;
déterminer, en tant que point A3, un point qui est un centre de gravité du site de liaison de la molécule cible et qui est différent de A1 ;
déterminer, en tant que point A4, un point qui est une coordonnée d'un atome lourd arbitraire de la molécule candidate médicamenteuse et qui est différent de A2 ;
déterminer, en tant que variable collective, un angle dièdre formé par un plan X formé par les points A1, A2 et A3 et un plan Y formé par les points A1, A2 et A4 ; et
rechercher la structure de liaison stable entre la molécule cible et la molécule candidate médicamenteuse en utilisant la variable collective utilisant la métadynamique,
dans lequel les vecteurs A1A2 et A2A3 sont linéairement indépendants du vecteur A2A4.
